# EUROPEAN PATENT APPLICATION

(11) **EP 4 772 627 A1**
(43) Date of publication of application: **08.07.2026**
(21) Application number: 23955857.0
(22) Date of filing: 31.10.2023
(51) Int. Cl.: C12N 15/11, C12N 15/10, C12N 9/00

(54) **TRNA AND BIOSYNTHESIS METHOD THEREFOR**

(30) Priority: 16.10.2023 CN 202311335092
(71) Applicant: Jilin Asymchem Laboratories Co., Ltd., Dunhua, Jilin 133700 (CN); Tianjin Asymchem Pharmaceuticals Co., Ltd., Tianjin 300462 (CN)
(72) Inventor: HONG, Hao, Morrisville North Carolina 27560 (US); JAMES, Gage, Morrisville North Carolina 27560 (US); ZHANG, Na, Tianjin 300457 (CN); JIAO, Xuecheng, Tianjin 300457 (CN); LIU, Fang, Tianjin 300457 (CN); MA, Cuiping, Tianjin 300457 (CN); PANG, Huining, Tianjin 300457 (CN); JIA, Xu, Tianjin 300457 (CN); WANG, Zhaoshuai, Tianjin 300457 (CN); GENG, Yuhan, Tianjin 300457 (CN); CUI, Lixin, Tianjin 300457 (CN); ZHU, Wenxuan, Tianjin 300457 (CN); ZHAO, Xiaolan, Tianjin 300457 (CN)
(74) Representative: Dehns Germany Partnerschaft mbB
(86) International application number: PCT/CN2023/128823
(87) International publication number: WO 2025/081539

(57) **Abstract**

Provided is tRNA and a method for biosynthesizing a tRNA thereof. The method for the tRNA includes: ligating the 3' end and 5' end of different substrates to form a phosphodiester bond with an RNA ligase, thereby forming the tRNA, where the number of the substrates is ≥2; and the RNA ligase includes any one or more enzymes in the RNA ligase families Rnl1, Rnl2, Rnl3 and Rnl5. The present disclosure can solve the problem in the prior art of low tRNA synthesis yields, and is suitable for the field of tRNA synthesis.

## Description

This application is based upon and claims priority to Chinese Patent Application No. 202311335092.4 filed on October 16, 2023, the disclosure of which is hereby incorporated by reference in its entirety.

### Technical Field

The present disclosure relates to the field of tRNA synthesis, and specifically, to tRNA and a method for biosynthesizing a tRNA thereof.

### Background

tRNA is a ribonucleic acid molecule composed of 70 to 120 nucleotides, serving as an adapter molecule in protein synthesis. There are over 100 types of tRNA molecules, each capable of carrying an amino acid and transporting same to the ribosome for use in protein synthesis. The secondary structure of tRNA is an inverted cloverleaf configuration. One end has a 3'-OH structure capable of ligating amino acids, and the other end has an anticodon that can form complementary base pairing with the codon on mRNA. RNA strands form local double helix structures through self-folding and complementary sequence pairing, known as stems or arms. Unpaired bases between two fragments (double helix structures) remain single-stranded, forming a loop. The stem and loop form a stem-loop structure, also known as a hairpin structure. Generally, if the RNA folding ratio is higher, the structure is more stable. tRNA not only plays a vital role within living organisms, but research has also demonstrated its potential for treating certain diseases caused by genetic mutations. For example, the 2021 Science paper titled "RNA overexpression rescues peripheral neuropathy caused by mutations in tRNA synthetase" revealed that insufficient intracellular tRNA supply due to genetic mutations leads to Charcot-Marie-Tooth disease (CMT). Increasing tRNAGly levels may hold promise as a potential therapeutic approach. Therefore, tRNA therapy may become an effective approach for treating human diseases in the future.

Currently, RNA synthesis is primarily achieved through solid-phase synthesis. However, the length of RNA obtained through solid-phase synthesis is limited. Typically, the tRNA, which is 70-120 nucleotides in length, cannot be synthesized through solid-phase synthesis. Currently, research on tRNA synthesis is extremely limited. The primary synthetic route originates from the intracellular synthesis of natural tRNA, followed by extraction to obtain tRNA. The route is extremely-low in yield and is prone to contamination by nucleases.

### Summary

The present disclosure is mainly intended to provide tRNA and a method for biosynthesizing a tRNA thereof, so as to solve the problem of difficulty in synthesizing tRNA and low yield in the prior art.

In order to implement the above objective, according to a first aspect of the present disclosure, a method for biosynthesizing a tRNA is provided. The method includes: ligating the 3' end and 5' end of different substrates to form a phosphodiester bond with an RNA ligase, thereby forming the tRNA, where the number of the substrates is ≥2; and the RNA ligase includes any one or more enzymes in the RNA ligase family Rnl1, Rnl2, Rnl3 or Rnl5.

Further, the substrates include one or more non-natural nucleotides, and the tRNA includes non-natural tRNA.

Further, the RNA ligase includes: one or more of the RNA ligases shown in SEQ ID NO: 1, SEQ ID NO: 2, SEQ ID NO: 3, SEQ ID NO: 4, SEQ ID NO: 5, SEQ ID NO: 6, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22 or SEQ ID NO: 23; or a protein having more than 70% identity with any of the RNA ligases shown in SEQ ID NO: 1 to SEQ ID NO: 6, or SEQ ID NO: 10 to SEQ ID NO: 23 and having an RNA ligase activity.

Further, the non-natural tRNA includes a stem-loop structure and a complementary region, and a ligation site of the substrates is located at the stem-loop structure and/or the complementary region.

Further, the 3' ends of the substrates are hydroxyl groups.

Further, the substrates include a first substrate and other substrates, where the 5' end of the first substrate forms the 5' end of the non-natural tRNA, and the 5' ends of the other substrates are phosphate groups.

Further, the number of the non-natural nucleotides in the substrates is ≥2.

Further, the nucleotides at the 5' ends and/or 3' ends of the substrates are the non-natural nucleotides.

Further, the substrates consist of the non-natural nucleotides.

Further, the number of the substrates is 2-6; and the length of each of the substrates is 15-60 nt.

Further, the method includes: substrate annealing and substrate ligation; the substrate annealing includes: the substrates are dissolved in an annealing buffer to form an annealing system, the annealing system is annealed according to an annealing program, where the annealing program includes: the annealing system is heated to 90 °C and held for 1 min, and then the temperature is lowered to 20 °C with a cooling rate of 0.1 °C per minute and incubation is performed for 10 min to obtain an annealing product; and the substrate ligation includes: the annealing product, the RNA ligase, and the ATP are prepared into a ligation system for the substrate ligation.

Further, the annealing buffer includes: 1 mM-10 M of NaCl, 1 mM-10 M of Tris-HCl at pH 4-10 and 0.1 mM-10 M of EDTA; a reaction temperature for the substrate ligation is 0-70 °C, and a reaction time is 10 min-100 h; and a concentration of the annealing product is 10 µM-2 mM, a concentration of the RNA ligase is 0.02-4 mg/mL, and a molar ratio of the ATP to the annealing product is 1-10: 1.

Further, the ligation system further includes MgCl₂ and dithiothreitol, where a concentration of MgCl₂ is 0.1 mM-200 M, and a concentration of dithiothreitol is 0.1 mM-10 M.

Further, the non-natural nucleotide includes a ribonucleotide having one or more of the following modifications: a ribose position 2' modification, a ribose skeleton modification, a base modification or a phosphate skeleton modification; the ribose position a 2' modification includes a 2'-methoxy modification, a 2'-fluoro modification, a 2'-methoxyethyl modification, a 2'-FANA modification, a locked nucleic acid modification or a hexitol nucleic acid modification; the ribose skeleton modification includes replacing the ribose in the nucleotide with ribuloNA, TNA, tPhoNA or dXNA; the base modification includes a denitrified adenine C7 modification, a denitrified guanosine C7 modification, a cytosine C5 modification, or a uridine C5 modification; and the phosphate skeleton modification includes a PS modification.

In order to implement the above objective, according to a second aspect of the present disclosure, non-natural tRNA is provided. The non-natural tRNA includes the tRNA obtained through preparation using the above method.

Further, the above tRNA includes the non-natural tRNA obtained through preparation using the above method.

With the technical solutions of the present disclosure, the tRNA is obtained through biosynthesis with any one or more enzymes in the RNA ligase families Rnl1, Rnl2, Rnl3 and Rnl5. With the method, the problem of low tRNA yield in the prior art can be solved.

### Brief Description of the Drawings

The drawings, which form a part of the present application, are used to provide a further understanding of the present disclosure. The exemplary embodiments of the present disclosure and the description thereof are used to explain the present disclosure, but do not constitute improper limitations to the present disclosure. In the drawings:
Fig. 1 is a schematic structural diagram of non-natural tRNA according to an embodiment of the present disclosure.
Fig. 2 is a PAGE electrophoresis result diagram of ligation products of a substrate 1 and a substrate 2 according to Embodiment 2 of the present disclosure.
Fig. 3 is a UPLC result diagram of ligation products of a substrate 3 and a substrate 4 according to Embodiment 3 of the present disclosure.
Fig. 4 is a UPLC result diagram of ligation products of a substrate 3 and a substrate 4 according to Embodiment 4 of the present disclosure.
Fig. 5 is a PAGE electrophoresis result diagram of ligation products of a substrate 9 and a substrate 10 according to Embodiment 6 of the present disclosure.
Fig. 6 is a UPLC result diagram of ligation products of a substrate 11 and a substrate 12 according to Embodiment 7 of the present disclosure.

### Detailed Description of the Embodiments

It is to be noted that the embodiments in the present application and the features in the embodiments may be combined with one another without conflict. The present disclosure will be described below in detail with reference to the embodiments.

As mentioned in the Background, research on tRNA synthesis remains extremely limited. The primary synthetic route originates from the intracellular synthesis of natural tRNA, followed by extraction to obtain tRNA. The route is extremely low in yield and is prone to contamination by nucleases. The inventor in the present application attempts to explore a method for biosynthesizing a tRNA, achieving the efficient preparation of the tRNA, thereby proposing a series of protective solutions of the present application.

In a first typical implementation of the present application, a biosynthetic method for tRNA is provided. The method includes: the 3' end and 5' end of different substrates are ligated to form a phosphodiester bond with an RNA ligase, thereby forming the tRNA, where the number of the substrates is ≥2; and the RNA ligase includes any one or more enzymes in the RNA ligase families Rnl1, Rnl2, Rnl3 or Rnl5.

In a preferred embodiment, the substrates include one or more non-natural nucleotides, and the tRNA includes a non-natural tRNA.

During the preparation of nucleic acid drugs using the tRNA, since natural RNA is susceptible to recognition and enzymatic cleavage by intracellular nucleases, most nucleic acid drugs use artificial, non-natural modifications to improve their resistance to the nucleases while improving specificity and reducing side effects. There are no reports in the prior art regarding the synthesis of non-natural tRNA. With the method of the present application, the natural tRNA can be synthesized, and the synthesis of the non-natural tRNA can also be realized.

In a preferred embodiment, the RNA ligase includes one or more of the RNA ligases shown in SEQ ID NO: 1, SEQ ID NO: 2, SEQ ID NO: 3, SEQ ID NO: 4, SEQ ID NO: 5, SEQ ID NO: 6, SEQ ID NO: 10, SEQ ID NO: 11, SEQ ID NO: 12, SEQ ID NO: 13, SEQ ID NO: 14, SEQ ID NO: 15, SEQ ID NO: 16, SEQID NO: 17, SEQ ID NO: 18, SEQ ID NO: 19, SEQ ID NO: 20, SEQ ID NO: 21, SEQ ID NO: 22, and SEQ ID NO: 23; or an protein that has more than 70%, preferably, more than 75%, more than 80%, more than 85%, more than 90%, more than 95%, more than 98%, more than 99%, more than 99.5%, or more than 99.9% identity to any one of the RNA ligases shown in SEQ ID NO: 1-SEQ ID NO: 6 or SEQ ID NO: 10-SEQ ID NO: 23, and a protein having an RNA ligase activity.

In the above method, with the above RNA ligase, substrates containing non-natural nucleotides can be ligated and form the tRNA having a higher-order structure. In the above method, since the target tRNA has a relatively complex higher-order structure and the target product is non-natural tRNA containing a chemical modification, the inventor screens the above RNA ligases having catalytic activity from numerous RNA ligases. The above RNA ligase has the activity of catalyzing the ligation of RNA fragments containing the non-natural nucleotides, and also has an active pocket matching the structure of the tRNA, such that the ligation of nicks between substrate RNA fragments in the tRNA using a phosphodiester bond can be catalyzed, so as to form the non-natural tRNA with a higher-order structure.

Identity in the present application refers to "identity" between amino acid sequences or nucleic acid sequences, that is, the total of ratios of the amino acid residues or nucleotides of the same type in the amino acid sequences or nucleic acid sequences. The identity of the amino acid sequences or nucleic acid sequences may be determined with comparison programs such as a Basic Local Alignment Search Tool (BLAST), FASTA, etc.

Active sites, active pockets, active mechanisms, protein structures, etc. of proteins having more than 70%, 75%, 80%, 85%, 90%, 95%, 99% (e.g., more than 85%, 86%, 87%, 88%, 89%, 90%, 91%, 92%, 93%, 94%, 95%, 96%, 97%, 98%, 98.5%, 99%, 99.5%, 99.6%, 99.7%, 99.8%, or even more than 99.9%) identity and having the same functions are probably the same as the protein provided the sequence in a).

As used herein, the amino acid residues are abbreviated below: Alanine (Ala; A), Asparagine (Asn; N), Aspartic acid (Asp; D), Arginine (Arg; R), Cysteine (Cys; C), Glutamate (Glu; E), Glutamine (Gln; Q), Glycine (Gly; G), Histidine (His; H), Isoleucine (Ile; I), Leucine (Leu; L), Lysine (Lys; K), Methionine (Met; M), Phenylalanine (Phe; F), Proline (Pro; P), Serine (Ser; S), Threonine (Thr; T), Tryptophan (Trp; W), Tyrosine (Tyr; Y), and Valine (Val; V).

For rules of substitution, replacement, etc., if amino acids are similar in nature, replacements have similar effects. For example, in the homologous protein, conservative amino acid replacements may occur. "Conservative amino acid replacement" includes, but is not limited to the followings.

Hydrophobic amino acids (Ala, Cys, Gly, Pro, Met, Val, Ile, and Leu) are substituted by other hydrophobic amino acids.

Hydrophobic amino acids with bulky side chains (Phe, Tyr, and Trp) are substituted by other hydrophobic amino acids with bulky side chains.

Amino acids with positively charged side chains (Arg, His, and Lys) are substituted by other amino acids with positively charged side chains.

Amino acids with polarized and uncharged side chains (Ser, Thr, Asn, and Gln) are substituted by other amino acids with polarized and uncharged side chains.

A person skilled in the art may also make conservative substitutions of the amino acids according to amino acid substitution rules known to person skilled in the art, such as a "blosum62 scoring matrix" in the prior art.

In a preferred embodiment, the non-natural tRNA includes a stem-loop structure and a complementary region, and a ligation site of the substrates is located at the stem-loop structure and/or the complementary region.

In a preferred embodiment, the 3' ends of the substrates are hydroxyl groups.

In a preferred embodiment, the substrates include a first substrate and other substrates, where the 5' end of the first substrate forms the 5' end of the non-natural tRNA, and the 5' ends of the other substrates are phosphate groups.

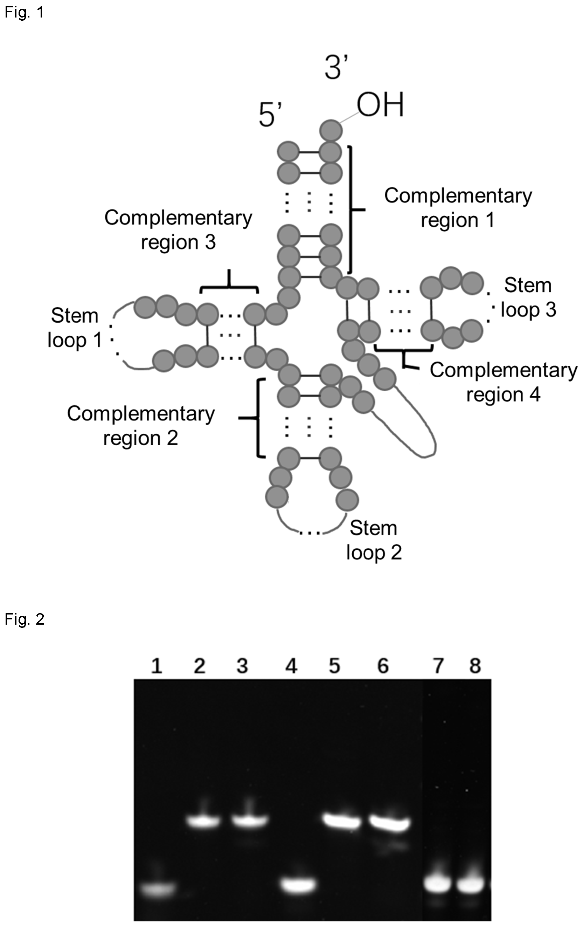

Fragments in which bases can be complementary to each other, exist between or in the above substrates. During a biosynthesis reaction, these complementary base pairing fragments bind to each other to form the complementary region of the non-natural tRNA (including a complementary region 1, a complementary region 2, a complementary region 3, and a complementary region 4). Following the completion of complementary base pairing, the higher-order structure of the non-natural tRNA is formed initially. Different substrates are ligated by hydrogen bonds formed through complementary base pairing. The head and tail bases of different substrates are not ligated with each other, resulting in a nick. The above nick, which is a ligation site of the substrate, is located in the stem-loop structure (including a stem loop 1, a stem loop 2, and a stem loop 3) and/or the complementary region of the non-natural tRNA. The active pocket of the above RNA ligase can match a three-dimensional structure of the non-natural tRNA. The catalytic activity is not affected by the three-dimensional structure. The substrates are catalytically ligated by the phosphodiester bond, so as to repair the nicks located on the non-natural tRNA. A schematic structural diagram of the above non-natural tRNA is shown in Fig. 1.

In a preferred embodiment, the number of the non-natural nucleotides in the substrates is ≥2. Preferably, the nucleotides at the 5' ends and/or 3' ends of the substrates are the non-natural nucleotides. Preferably, the substrates consist of the non-natural nucleotides.

Xeno-nucleic acids (XNA) are a class of nucleic acid molecules having non-natural backbones or nucleic acid bases. A non-natural ribonucleotide is a ribonucleotide having a non-natural backbone or nucleic acid base. The tRNA prepared by ligating substrates containing non-natural ribonucleotides includes non-natural ribonucleotides and belongs to non-natural tRNA.. When the 3' terminal base of a substrate donor and/or the 5' terminal base of a substrate receptor are non-natural bases, although a nucleotide at a junction is different from a natural nucleotide and belongs to an artificially-created non-natural nucleotide, the above RNA ligase can also achieve ligation activity.

In a preferred embodiment, the number of the substrates is 2-6, including 2, 3, 4, 5, and 6. If the number of the substrates is too many, ligation efficiency and substrate conversion rate are relatively low.

In a preferred embodiment, the length of each of the substrates is 15-60 nt, including, but not limited to, 15, 20, 25, 30, 35, 40, 45, 50, 55, and 60 nt.

In a preferred embodiment, reaction conditions for the method include: substrate annealing and substrate ligation; the substrate annealing includes: the substrates are dissolved in an annealing buffer to form an annealing system, the annealing system is annealed according to an annealing program, where the annealing program includes: the annealing system is heated to 90 °C and held for 1 min, and then the temperature is lowered to 20 °C with a cooling rate of 0.1 °C per minute and incubation is performed for 10 min to obtain an annealing product; and the substrate ligation includes: the annealing product, the RNA ligase, and the ATP are prepared into a ligation system for the substrate ligation.

Preferably, the annealing buffer includes: 1 mM-10 M of NaCl, 1 mM-10 M of Tris-HCl at pH 4-10 and 0.1 mM-10 M of EDTA; and more preferably, the annealing buffer includes: 10-500 mM NaCl, 1-1000 mM Tris-HCl, pH 5-9, and 0.1-10 mM EDTA. Preferably, a reaction temperature for the substrate ligation is 0-70 °C, and a reaction time is 10 min to 100 h; and more preferably, the reaction temperature for the substrate ligation is 0-50 °C, and the reaction time is 0.2-72 h. Preferably, a concentration of the annealing product is 10 µM-2 mM, a concentration of the RNA ligase is 0.02-4 mg/mL, and a molar ratio of the ATP to the annealing product is 1-10: 1. Preferably, the ligation system further includes MgCl₂ and dithiothreitol. Preferably, a concentration of MgCl₂ is 0.1 mM-200 M, and a concentration of dithiothreitol is 0.1 mM-10 M. More preferably, the concentration of MgCl₂ is 1-20 M, and the concentration of dithiothreitol is 0.1-10 mM.

In the annealing buffer, the concentration of NaCl includes, but is not limited to, 1 mM, 2 mM, 3 mM, 5 mM, 10 mM, 20 mM, 50 mM, 100 mM, 200 mM, 500 mM, 1 M, 2 M, 3 M, 5 M, 7 M, or 10 M. The concentration of Tris-HCl includes, but is not limited to, 1 mM, 2 mM, 3 mM, 5 mM, 10 mM, 20 mM, 50 mM, 100 mM, 200 mM, 500 mM, 1 M, 2 M, 3 M, 5 M, 7 M, or 10 M, and the pH includes, but is not limited to, 4, 4.5, 5, 5.5, 6, 6.5, 7, 7.5, 8, 8.5, 9, 9.5, or 10. The concentration of EDTA includes, but is not limited to, 1 mM, 2 mM, 3 mM, 5 mM, 10 mM, 20 mM, 50 mM, 100 mM, 200 mM, 500 mM, 1 M, 2 M, 3 M, 5 M, 7 M, or 10 M. The concentration of MgCl₂ includes, but is not limited to, 0.1 M, 0.2 M, 0.3 M, 0.5 M, 1 M, 2 M, 3 M, 5 M, 10 M, 20 M, 30 M, 40 M, 50 M, 70 M, 100 M, 150 M, or 200 M. The concentration of dithiothreitol includes, but is not limited to, 0.1 mM, 0.2 mM, 0.3 mM, 0.5 mM, 1 mM, 2 mM, 3 mM, 5 mM, 10 mM, 20 mM, 50 mM, 100 mM, 200 mM, 500 mM, 1 M, 2 M, 3 M, 5 M, 7 M, or 10 M.

The reaction temperature includes, but is not limited to, 0 °C, 10 °C, 20 °C, 30 °C, 40 °C, 50 °C, 60 °C, or 70 °C. The reaction time includes, but is not limited to, 10 min, 20 min, 30 min, 1 h, 2 h, 3 h, 4 h, 5 h, 7 h, 10 h, 15 h, 20 h, 30 h, 40 h, 50 h, 60 h, 70 h, 80 h, 90 h, or 100 h.

**In** a preferred embodiment, the non-natural nucleotide includes a ribonucleotide having one or more of the following modifications: a ribose position 2' modification, a ribose skeleton modification, a base modification or a phosphate skeleton modification; preferably, the ribose position a 2' modification includes a 2' methoxy modification, a 2'-fluoro modification, a 2'-methoxyethyl modification, a 2'-FANA modification, a locked nucleic acid modification or a hexitol nucleic acid modification; the ribose skeleton modification includes replacing the ribose in the nucleotide with ribuloNA, TNA, tPhoNA or dXNA; the base modification includes a denitrified adenine C7 modification, a denitrified guanosine C7 modification, a cytosine C5 modification, or a uridine C5 modification; and the phosphate skeleton modification includes a PS modification.

The locked nucleic acid modification is 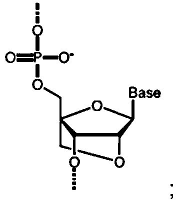 the hexitol nucleic acid modification is 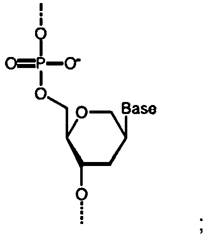 2'-methoxyethyl modification is 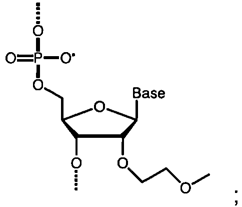 2'-fluoro modification is 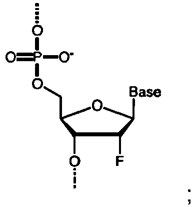 2'-FANA is 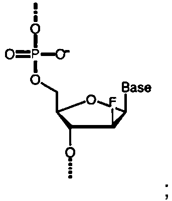 ribuloNA is 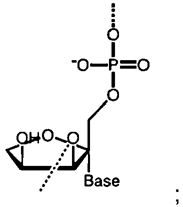 TNA is 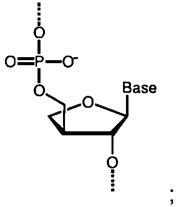 tPhoNA is 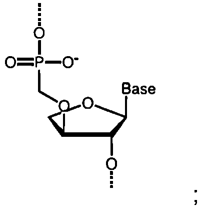 **dXNA** is 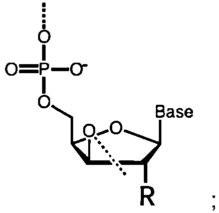 and PS modification is 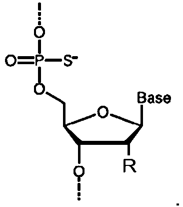 Bases in the above structures all represent bases, R represents hydroxyl, hydrogen, methoxy, methoxyethyl, or fluoro modification. For the above modification of non-natural nucleotides, refer to literature: Duffy K, Arangundy-Franklin S, Holliger P. Modified nucleic acids: replication, evolution, and next-generation therapeutics[J].BMC Biology, 2020, 18(1): 112.

In a second typical implementation of the present application, tRNA is provided. The tRNA includes the tRNA obtained through preparation using the above method.

In a preferred embodiment, the above tRNA includes the non-natural tRNA obtained through preparation using the above method.

Sequences, sources, and families of the ligases used in the present application are as follows.
Ligase L-1, SEQ ID NO: *1, Escherichia phage* JN02, RNA ligase 1 (RNA ligase family Rnl1): 
HMEKLYYNLLSLCKSSSDRKFFYSDDVSPIGKKYRIFSYNFASYSDWLLPDALEC
RGIMFEMDGETPVRIASRPMEKFFNLNENPFTLSINLDDVKYLMTKEDGSLVSTYLDG
GTVRFKSKGSIKSDQAVSATSILLDIDHKNLADRLLELCNDGFTANFEYVAPTNKIVLTY
PEKRLILLNIRDNNTGEYIEYDDIYLDPVFRKYLVDRFEVPEGDWTSDVKSSTNIEGYVA
VMKDGSHFKLKTDWYVALHTTRDSISSPEKLFLAIVNGASDDLKAMYADDEFSFKKVE
LFEKAYLDFLDRSFYICLDTYDKHKGKDRKTYAIEAQAVCKGAQTPWLFGIIMNLYQGG
SKEQMMTALESVFIKNHKNFIPEGY.
Ligase L-2, SEQ ID NO: 2, *Bacteriophage* AR1, RNA ligase 1: 
MQELFNNLMELCKDSQRKFFYSDDVSASGRTYRIFSYNYASYSDWLLPDALECR
GIMFEMDGEKPVRIASRPMEKFFNLNENPFTMNIDLNDVDYILTKEDGSLVSTYLDGDE
ILFKSKGSIKSEQALMANGILMNINHHQLRDRLKELAEDGFTANFEFVAPTNRIVLAYQE
MKIILLNIRENETGEYISYDDIYKDAALRPYLVERYEIDSPKWVEEAKNAENIEGYVAVMK
DGSHFKIKSDWYVSLHSTKSSLDNPEKLFKTIIDGASDDLKAMYADDEYSYRKIEAFET
TYLKYLDRALFLVLDCHNKHCGKDRKTYAMEAQGVAKGAGMDHLFGIIMSLYQGYDS
QEKVMCEIEQNFLKNYKKFIPEGY.
Ligase L-3, SEQ ID NO: 3, *Bacteriophage dhaeg,* RNA ligase 2 (RNA ligase family Rnl2): 
MFKKYSSLENHYNSKFIEKLRTNGLTGGEWVAREKIHGTNFSLIIERDAVTCAKRT
GPILPAEDFYGYEIVLKNYADSIKSIQDIMETSAAVSYQVFGEFAGTGIQKNVDYGDKDF
YVFDIIVTTESGDVTYVDDYMMESFCKTFKFKMAPLLGRGKFEDLIKLPNDLDSVLPDY
NFTVDNVGLAEANAHVWNAEAKGEVFTAEGYVLKPCYPLWLPNGNRVAIKCKNSKFS
EKKKTDKPIKVAVVLSQDDLDLLQQFTDYVTVNRINNVISKIGEVSPKDFGKVMGLTVQ
DILEEAAREELELTDAENPVEVKKQLIECVKDTLRAVWIELVSK.
Ligase L-4, SEQ ID NO: 4, *Vibrio phage,* RNA ligase 1: 
MSFVKYTSLENSYRQAFVDKCDMLGVREWVALEKIHGANFSFIVEFKPNEAQDG
AEFTVTPAKRTSTIGANVMGDYDFYGCTSVVEAHTAKMEAISNWLWARGIINVGETIIV
YGELAGKGVQKEVNYGDKDFWAYDILLPETGKFLDWDVVLEACEFAKVKTTHEIARGT
 
LDELLRIDPLFRSFHTPADVDGDNVAEGFVVKQLRNEKRLHNGSRAILKVKNDKFKEK
KNKAGKTPRAAVVLTEEQEKLHAAFSCYLTENRLRNVLSKLETVTQKQFGMISGLFIKD
AKDEFERDELNETAIARDDWDVIKRSLTNIANEILRKNWLDILDGNF.
Ligase L-5, SEQ ID NO: 5, *Archaea,* RNA ligase 3 (RNA ligase family Rnl3): 
MVSSVYKEILVKLGLTEDRIETLEMKGGIIEDEFDGIRYVRFKDSAGKLRRGTVVID
EEYVIPGFPHIKRIINLRSGIRRIFKRGEFYVEEKVDGYNVRVVMYKGKMLGITRGGFIC
PFTTERIPDFVPQEFFKDNPNLILVGEMAGPESPYLVEGPPYVKEDIQFFLFDVQEIKTG
RSLPVEERLKIAEEYGINHVEVFGKYTKDDVDELYQLIERLSKEGREGIIMKSPDMKKIV
KYVTPYANINDIKIGARVFYELPPGYFTSRISRLAFYLAEKRIKGEEFERVAKELGSALLQ
PFVESIFDVEQEEDIHELFKVRVKRIETAYKMVTHFEKLGLKIEIVDIEEIKDGWRITFKRL
YPDATNEIRELIGGKAFVD.
Ligase L-6, SEQ ID NO: 6, *fungus Deinococcus radiodurans,* RNA ligase 5 (RNA ligase family Rnl5): 
MAERQVIKERAQLFPHPNAERLELCKVGTFQLVVRKGEYRDGDPIVIAPEKAVLP
PQLAGLYTNADTGASYLHGAEKNRVGSVRLRGEVSQGVILPLDGLEDAPFGEDLAER
LGITFWEPPVPVSMAGEVEPRPPAQHYKHHDVEQFGIYVSEFASGEEVMVTEKLHGT
QGVYFRTAEGRWLVTSKGLSRGGLTLREAASNVYWQAARNSNLFAEADAAFSGGEV
QIFGEVVPVQKGFSYGQHKPTVFVFKVVYDGLRLPRRDWPQWVLDHAVPVLYEGPF
DEATVRKLRGGLETVSGKGLHIREGVVVAPKVPRFAADGSDLSVKLISDAYAKKETGE
EYS.
Ligase L-7, SEQ ID NO: 7, *Trypanosoma brucei brucei,* RNA ligase 2: 
MVGGDGSIFERYTEIDNSNERRINALKGCGMFEDEWIATEKVHGANFGIYSIEGE
KMIRYAKRSGIMPPNEHFFGYHILIPELQRYVTSIREMLCEKQKKKLHVVLINGELFGGK
YDHPSVPKTRKTVMVAGKPRTISAVQTDSFPQYSPDLHFYAFDIKYKETEGGDYTTLV
YDEAIELFQRVPGLLYARAVIRGPMSKVAAFDVERFVTTIPPLVGMGNYPLTGNWAEGL
VVKHSRLGMAGFDPKGPTVLKFKCTAFQEISTDRAQGPRVDEMRNVRRDSINRAGVQ
 
LPDLESIVQDPIQLEASKLLLNHVCENRLKNVLSKIGTEPFEKEEMTPDQLATLLAKDAL
KDFLKDTEPSIVNIPVLIRKDLTRYVIFESRRLVCSQWKDILKRQSPDFSE.
Ligase L-8, SEQ ID NO: 8, *Acidobacteria bacterium,* RNA ligase 1: 
MKQMYDNLKALTQKNDMFFSSIQTTPSNKPVEIFSYHIASYSDWLEPDAIECRGIM
FDITDKENPVILSRPMEKFFNLGENPLALPEDVLPLVKTIEEKRDGSLISTYLDDGKLYT
KSKGSLYSDQANDSNRFLMRSENKDFKQALKTLAELGYTANMEYTSPKNQIVLKYEK
QELIVLNIRNTETGDYLTLDDLAELDAENPEHTIYTHIAERFVDYEEVDGMSDEDVERIR
DMKGIEGYVLVGQNQRVKLKTDWYVNLHRLKDNINNNRRLVESVVESRTDDLRQLFE
HDQGSIDKIKEFEQYVLSKIKSCLAEVRTVYERVKHLDRKHYAINAQRYVDYTPLFSVIM
RQFDDYNEENVVENIKQIALKNVDDFIPMHYK.
Ligase L-9, SEQ ID NO: 9, *fungus Magnaporthe grisea,* RNA ligase 5: 
MEGDSPVVAVTSPTIPTDSTLAVSPTAEKAASTESFSAPAVRKLVTLRRISRIRPVK
NAKLPKSKYVAITVDGWEVVVNAKRDQRRFREGQLVVFFQIDSFVPENDGNFWEEMS
TSAELFQERRGFRIGTLRIGESSLISQGRVFHLGEYPRVEEVVAELQKVHGDKDGLEV
AMQRSFDDVLGVVKWDVPEEHKGAHYGPPPPMIPRTALNRIQDVTQQLWERHAATR
VQITEKLDSCPISIYFVRNDSPFAKCLPALHDFAGKPDAQRRQQLCVFPHGRVGVCA
NRLEYVCAPGQRFWAVVERLGIPGRLAREGRTAVVQGELVAPDRGGPGGFYAFSLWT
EGGKHGGFDGEETQRAALRRMGVPLVPLVGYRAIGEFASSTGEMLAKADGRGFRGQ
LREGFVLKNVKENIWVKVISNAWLEWYEE.
Ligase L-10, SEQ ID NO: 10, *Klebsiella phage* KP15, RNA ligase 2: 
MFKKYSSLTNHYEGKFINGVIMNGLTGGVWVAREKIHGANFSFITDDGITVTPAKR
TDVVKPAEDFYGCSAVVAKYSPGIRKMWETLKKTGTYDDLVIQVYGEFAGRGVQKDV
DYGEKDFYVFDIRVNGEFLPDNLCSLISRSHGLKMAPLLGYGTFEEIKELPITFESVVNK
ANSGIGSDNTVYGEFVYPIMDVEEGNIAEGFVMKPVSPAFMPNGERVAIKCKTTKFTE
KKAKKATRFNAPVSLSEKDKNQLDEFVCYLTENRVKNVLSKLDLASITAKDFGRIMGLT
VQDAIEEISRNHGPFIEQFEDPAMAKKLFVTEAQNMIRPVWGKILNHEF.
Ligase L-11, SEQ ID NO: 11, *Vibrio phage,* RNA ligase 1: 
MTTQELYNHLMTLTDDAEGKFFFADHISPLGEKLRVFSYHIASYSDWLLPGALEAR
GIMFQLDEQDKMVRIVSRPMEKFFNLNENPFTMDLDLTTTVQLMDKADGSLISTYLTG
ENFALKSKTSIFSEQAVAANRYIKLPENRDLWEFCDDLTQAGCTVNMEWCAPNNRIVL
EYPEAKLVILNIRDNETGDYVSFDDIPLPALMRVKKWLVDEYDPETAHADDFVEKLRAT
KGIEGMILRLANGQSVKIKTQWYVDLHSQKDSVNVPKKLVTTILNNNHDDLYALFADDK
PTIDRIREFDSHVSKTVSASFHAVSQFYVKNRHMSRKDYAIAGQKTLKPWEFGVAMIAY
QNQTVEGVYEALVGAYLKRPELLIPEKYLNEA.
Ligase L-12, SEQ ID NO: 12, *Bacteriophage,* RNA ligase 2: 
MFKKYSSLENHYNSKFIEKLYSLGLTGGEWVAREKIHGTNFSLIIERDKVTCAKRT
GPILPAEDFFGYEIILKNYADSIKAVQDIMETSAVVSYQVFGEFAGPGIQKNVDYCDKDF
YVFDIIVTTESGDVTYVDDYMMESFCNTFKFKMAPLLGRGKFEELIKLPNDLDSVVQD
YNFTVDHAGLVDANKCVWNAEAKGEVFTAEGYVLKPCYPSWLRNGNRVAIKCKNSKF
SEKKKSDKPIKAKVELSEADNKLVGILACYVTLNRVNNVISKIGEIGPKDFGKVMGLTVQ
DILEETSREGITLTQADNPSLIKKELVKMVQDVLRPAWIELVS.
Ligase L-13, SEQ ID NO: 13, *bacteria,* RNA ligase 2: 
MSFVKYTSLENSYRQAFVDKCDMLGVRDWVALEKIHGANFSFIVEFDGGYTVTP
AKRTSIIGATATGDYDFYGCTSVVEAHKEKVELVANFLWLNEYINLYEPIIIYGELAGKGI
QKEVNYGDKDFWAFDIFLPQREEFVDWDTCVAAFTNAEIKYTKELARGTLDELLRIDPL
FKSLHTPAEHEGDNVAEGFVVKQLHSEKRLQSGSRAILKVKNEKFKEKKKKEGKTPTK
LVLTPEQEKLHAEFSCYLTENRLKNVLSKLGTVNQKQFGMISGLFVKDAKDEFERDEL
NEVAIDRDDWNAIRRSLTNIANEILRKNWLNILDGNF.
Ligase L-14, SEQ ID NO: 14, *Vibrio phage* phi-ST2, RNA ligase 2: 
MNVQELYKNLMSLADDAEGKFFFADHLSPLGEKFRVFSYHIASYSDWLLPGALEA
RGIMFQLDDNDEMIRIVSRPMEKFFNLNENPFTMELDLTTTVQLMDKADGSLISTYLSG
ENFALKSKTSIFSEQAVAANRYIKKPENRDLWEFCDDCTQAGLTVNMEWCAPNNRIVL
EYPEAKLVILNIRDNETGDYVSFDDIPQSALMRVKQWLVDEYDPATAHEPDFVEKLRDT
KGIEGMILRLANGQSVKIKTQWYVDLHSQKDSVNVPKKLVTTILNGNHDDLYALFADDK
 
PTIERIREFDSHVTKTLTNSFNAVRQFYARNRHLARKDYAIAGQKVLKPWEFGVAMIAY

QKQTVEGVYESLVTAYLKRPELAIPEKYLNGV.
Ligase L-15, SEQ ID NO: 15, *Vibrio phage* JN02, RNA ligase 2: 
MFKKYSSLENHYNSKFIEKLYTNGLTTGVWVAREKIHGTNFSLIIERDNVTCAKRT
GPILPAEDFYGYEIVLKKYDKAIKAVQEVMESISTSVPVSYQVFGEFAGGGIQKGVDYG
EKDFYVFDIIINTESDDTYYMSDYEMQDFCNTFGFKMAPMLGRGTFDSLIMIPNDLDSV
LAAYNSTASEDLVEANNCVFDANVIGDNTAEGYVLKPCFPKWLSNGTRVAIKCKNSKF
SEKKKSDKPVKTQVPLTEIDKNLLDVLACYVTLNRVNNVISKIGTVTPKDFGKVMGLTV
QDILEETSREGIVLTSSDNPNLVKKELVRMVQDVLRPAWIELVS.
Ligase L-16, SEQ ID NO: 16, *Vibrio phage* JS98, RNA ligase 2: 
MFKKYSSLENHYNSKFIEKLRTNGLTGGEWVAREKIHGTNFSLIIERDAVTCAKRT
GPILPAEDFYGYEIVLKNYADSIKSVQHLIESINYQSYQIYGELAGPGIQKNVDYGDKDF
YVFDIRVTKEDGTESVLTDTLMEAFCIIHKFKVAPCLATGSFEDLIKLPNDFDSVIPDYNF
AVDNAGLTIANSTDFIPKVEGKVFTAEGFVLKPDIPTWLPNGNRVAIKCKNSKFSEKKKS
DKPIKAAVVLSQDDMDLMWQFTDYVTVNRINNVISKIGEVSKKDFGKVMGLTVQDILEE
AAREELELTDAENPVEVKKQLVECVKDTLRAVWIELVS.
Ligase L-17, SEQ ID NO: 17, *Trypanosoma brucei gambiense,* RNA ligase 2: 
MLRCLGVRHFRRTPLLFVGGDGSIFERYTEIDNSNERRINALKGCGMFEDEWIAT
EKVHGANFGIYSIEGEKMIRYAKRSGIMPPNEHFFGYHILIPELQRYVTSIREMLCEKQK
KKLHVVLINGELFGGKYDHPSVPKTRKTVMVAGKPRTISAVQTDSFPQYSPDLHFYAF
DIKYKETEGGDYTTLVYDEAIELFQRVPGLLYARAVIRGPMSKVAAFDVERFVTTIPPLV
GMGNYPLTGNWAEGLVVKHSRLGMAGFDPKGPTVLKFKCTAFQEISTDRAQGPRVD
EMRNVRRDSINRAGVQLPDLESIVQDPIQLEASKLLLNHVCENRLKNVLSKIGTEPFEK
EEMTPDQLATLLAKDAMKDFLKDTEPSIVNIPVLIRKDLTRYVIFESRRLVCSQWKDILK
RQSPDFSE.
Ligase L-18, SEQ ID NO: 18, *Bacteriophage RB69,* RNA ligase 1: 
MEKLYYNLLSLCKSSSDRKFFYSDDVSPIGKKYRIFSYNFASYSDWLLPDALECR
GIMFEMDGETPLRIASRPMEKFFNLNENPFTLSIDLNDVKYLMTKEDGSLVSTYLDGN
MVRFKGSIKSDQAASATSILLDINHKDLADRLLELCNDGFTANFEYVAPSNKIVLTYPEK
RLILLNIRDNNTGKYIEYDDIYLDPVFRKYLVDRFEAPEGDWVPGVKSSTNIEGYVAVM
KDGSHFKLKTDWYVALHTTRDSISSPEKLFLAIMNGASDDLKAMYADDEFSFKKVELF
EKAYLDFLDRSFYICLDAYDKHKGKDRKTYAIEAQAICKGAQSPWLFGIIMNLYQGGSK
EQMMTALESVFIKNHKNFIPEGY.
Ligase L-19, SEQ ID NO: 19, *Bacteriophage,* RNA ligase 1: 
MQELFNNLMELCKDSQRKFFYSDDVSASGRTYRIFSYNYASYSDWLLPDALECR
GIMFEMDGEKPVRIASRPMEKFFNLNENPFTMNIDLNDVDYILTKEDGSLVSTYLDGDE
ILFKSKGSIKSEQALMANGILMNINHHRLRDRLKELAEDGFTANFEFVAPTNRIVLAYQE
MKIILLNVRENETGEYISYDDIYKDATLRPYLVERYEIDSPKWIEEAKNAENIEGYVAVMK
DGSHFKIKSDWYVSLHSTKSSLDNPEKLFKTIIDGASDDLKAMYADDEYSYRKIEAFET
TYLKYLDRALFLVLDCHNKHCGKDRKTYAMEAQGVAKGAGMDHLFGIIMSLYQGYDS
QEKVMCEIEQNFLKNYKKFIPEGY.
Ligase L-20, SEQ ID NO: 20, *bacterial,* RNA ligase 3: 
MNSMNSDIPFDLIQERTGVPSSRLKVAFARGSLRLLESAGMQALLFKKPLGDLEA
GTVIYLGDETEVIRGFPKIRRTLLLSPTIQEHFRDRVAVEEKMNGYNVRIACLSSGETVA
LTRGGHVCPFTTRKAQELLDLSEFFREHPDLVICGEMIGRDNPYVSQDYPEVGPLGFR
VFDLREKNTNRPLPVEERRALLDSYGLPNVRLFGVYPIEEAASEVADIIRALGMAGREG
VVMKDPSMEVPPLKYTSSQAHARELAYAFSYPFDFGRPFFFSRVIREGFQAYELDESD
DETRERARRLGEAIIYPMLERIKSISAGEAAYEDTVIDVEDREAAEEFIRHLVRLGVSATL
ADYRDGRATIRRFYQSTTDRINNYLKGGLY.
Ligase L-21, SEQ ID NO: 21, *Klebsiella phage KP179,* RNA ligase 1: 
MLELYKNLMNLCESSEVAKFFYKDFTGPMDGKFRVFSYHYASYSEWLKPDALEC
RGIMFEMDGDTPIRIASRPMEKFFNLNENPLTMGIDISDVEYIMDKADGSLVSSYVDDG
YLYLKSKTSLYSDQARQASALLNSEEYSSLHQVILELTLDGYTVNMEFVSPNNRVVLAY
 
QEPQLFVLNVRNNTTGEYIKYDDLYANAKIRPYLINAYGISDPTTWVEGVRELEGVEGY
IAVLNTGQRFKVKTEWYSALHHTKDSITSNERLFASVVSANSDDLRSLFAGDEYAIKKIS
AFEQAYLDYLGKSLELCQSFYDEYRGRARKDYAIAAQKATVNQRHLFGVIMNMYEGT
VDVDKLLKDLERVFLKYWAGYVPKEYEKEIELSEE.
Ligase L-22, SEQ ID NO: 22, *Vibrio phage nt-1 rnlA,* RNA ligase 2: 
MNELYNNLMTLAESAEGKFFFADHLSPQGEKFRVFSYHIASYSDWLLPDALEARG
IMFQLDENDEMIRIVSRPMEKFFNLGENPFTMDLDLTTTVQLMDKADGSLISTYLTGDN
FALKSKTSIYSEQAVAANRYIKHVDNRDLWEFCDDCAAQNFTVNMEWCAPNNRIVLEY
TEPKLIILNIRDNETGQYVSFDDIPIGALTRIKKWLVDEYDPMTAHVDDFVETLKAKKGIE
GMILRLASGQSVKIKTTWYVDLHAQKDSVNSPKKLVTTILNKNHDDLYALFADDKPTIE
RIREFDDHVSKKVVESFHAVSQYYTKNRHLSRKDFAIAGQKALKPWEFGVAMIAYQNQ
TVEGVVTMLINAYLKRPELLIPEKYLSEV.
Ligase L-23, SEQ ID NO: 23, *Vibrio phage phi-ST2,* RNA ligase 2:

MSFVKYTSLENSYRQAFVDKCDMLGVKEWVALEKIHGANFSFIVEFKPSTEEVPG
EMSVTPAKRTSTIGANAMGDYDFYGCTSVVEAHIEKMQDISNWLFANDFIKNDETIIVY
GELAGKGIQKEVNYGDKDFWAYDILCPETGEFLDWDVVLKACKFAGVKTTHEIARGTL
DELLKIDPLFRSFHTPADVDSENVAEGFVVKQLKAEKRLHNGSRAILKVKNEKFKEKKN
KQGKTPRAKVVLTEEQEKLHAAFSCYLTENRLRNVLSKIGKVEAKQFGMVSGLFVKDA
KDEFERDERDEVAIPRDDWDVIKRSLVNVANEILRKNWLNIVDGTF.

The beneficial effects of the present application are further described in detail below with reference to specific embodiments.

### Embodiment 1 Purification and preparation of RNA ligase

A ligase protein was optimized, by a codon, as a host strain for recombinant expression in Escherichia coli; the optimized codon obtained target genes through synthesis; the target genes were respectively constructed onto a pET28a(+) expression vector with Ncol and Xhol as enzyme digestion sites; and there were 10 his tags on an N-end band. The recombinant plasmid containing a ligase gene was transformed into a cloning strain DH5α, and cultured overnight on an LB+1.5% agar culture medium containing 50 µg/mL of kanamycin, so as to obtain a monoclonal colony. The sequence of the monoclonal colony was confirmed through sequencing. The monoclonal colony was transferred into the liquid LB culture medium containing 50 µg/mL of kanamycin and cultured for 16 h at 37 °C; centrifugation was performed on the bacteria; and supernatant was discarded to obtain bacterial sludge. Plasmids were extracted from the obtained bacterial sludge with a plasmid extraction kit, the obtained plasmids were transformed into a BL21(DE3) expression strain, and overnight culture was performed on the LB+1.5% agar culture medium containing 50 µg/mL of kanamycin, so as to obtain the monoclonal colony. The obtained monoclonal colony was transferred into a 5 mL LB culture medium containing 50 µg/mL of kanamycin and cultured for 16 h at 37 °C, so as to obtain a seed solution. The seed solution was inoculated in the 500 mL LB culture medium (containing 50 µg/mL of kanamycin) according to an inoculation amount of 0.1%, and was cultured at 37 °C until OD = 0.6; 0.1M IPTG was added for induction; a culture temperature was regulated to 20 °C; and induction was performed for 16 h at 220 rpm, thereby obtaining expression-induced ligase Escherichia coli bacterial fluid. Centrifugation was performed on the bacterial fluid at 8000 rpm, and expression-induced Escherichia coli bacterial sludge was obtained after the supernatant was discarded. 10 mM of imidazole was added to the crude enzyme solution; an enzyme solution obtained through filtration using a 0.22 µm filter membrane was purified with a nickel column; desalination and solution exchange was performed, and then secondary purification was performed with a strong anion column; and the obtained enzyme solution was subjected to desalination and solution exchange and then stored in 30% glycerol.

### Embodiment 2 Catalytic synthesis of natural tRNA using RNA ligase

A substrate 1 and a substrate 2 were dissolved in an annealing buffer (100 mM NaCl, 10 mM Tris-HCl, pH 7.5, 1 mM EDTA) based on a final concentration of 200 µM, so as to perform annealing slowly. An annealing program included: the system was heated to 90 °C and held for 1 min, and then the temperature was lowered to 20 °C with a cooling rate of 0.1 °C per minute, and incubation was incubated for 10 min. Sequences of the substrate 1 and substrate 2 were shown in Table 1.

The annealed substrate 1 and substrate 2 were added to a clean reagent bottle to prepare a reaction system, causing a final volume to be 100 µL; and concentrations of the substrate 1 and substrate 2 were 100 µM, the concentration of ATP was 0.4 mM, the concentration of Tris-HCl (pH 7.5@25 °C) was 50 mM, the concentration of MgCl₂ was 10 mM, the concentration of DTT (dithiothreitol) was 1 mM, and the concentration of a ligase was 0.2 mg/mL. The reaction system underwent a reaction for 16 h at 16 °C. The obtained reaction system was held for 5 min at 80 °C to deactivate the ligase, a sediment was removed through centrifugation at 12000 rpm. A product was produced through mass spectrometry identification. Results showed that molecular weights of 23571±2 were detected in the reaction systems of the ligases L-1, L-2, L-3, L-4, L-5, L-6, L-11, L-18, L-19, L-20, L-21, L-22, and L-23, and a theoretical molecular weight was 23568±5. It indicated that a tRNA product was produced. No product molecular weight was detected in the reaction system of L-7, L-8, and L-9, indicating that no tRNA product was produced. PAGE gel detection was performed on a partial reaction system. An electropherogram was shown in Fig. 2. A lane 1 was a reaction system that was catalyzed using the ligase L-9, Lane 2 was a reaction system that was catalyzed using the ligase L-1, Lane 3 was a reaction system that was catalyzed using the ligase L-2, Lane 4 was a negative control group without adding the ligase, Lane 5 was a reaction system that was catalyzed using the ligase L-3, Lane 6 was a reaction system that was catalyzed using the ligase L-11, Lane 7 was a reaction system that was catalyzed using the ligase L-7, and Lane 8 was a reaction system that was catalyzed using the ligase L-8.

It was found that due to the relatively large fragment length, PAGE gel detection could not effectively distinguish the two substrates, and the substrate 1 and the substrate 2 eluted at the same position, resulting in a single nucleic acid band in the enzyme-free reaction system, that is, a mixed band of the substrate 1 and the substrate 2. Moreover, it was found that no new peaks were generated in the reaction system of L-7, L-8, and L-9, while new peaks were observed in the systems where product molecular weight was detected, indicating the feasibility of the experimental protocol. The nick site of the above ligation was located in the stem-loop structure of the tRNA..

**Table 1**

| Serial number | Sequence | 5' end | 3' end |
|---|---|---|---|
| Substrate 1 (SEQ ID NO: 24) | 5’-GUGUCCCUGCGGCAAUGG AUAGCCGAUUGGACUUCAA-3 | Hydroxyl group | Hydroxyl group |
| Substrate 2 (SEQ ID NO: 25) | 5'-P-AUUCAAAGGUUCCGGGU UCGAGUCCCGGGGGACGCG- 3' | Phosphate group | Hydroxyl group |

In the present application, the P represented a phosphate group on a C atom at position 5 of a 5' end nucleotide.

### Embodiment 3 Catalytic synthesis of non-natural tRNA using RNA ligase

A substrate 3 and a substrate 4 were dissolved in an annealing buffer (100 mM NaCl, 10 mM Tris-HCl, pH 7.5, 1 mM EDTA) based on a final concentration of 200 µM, so as to perform annealing slowly. An annealing program included: the system was heated to 90 °C and held for 1 min, and then the temperature was lowered to 20 °C with a cooling rate of 0.1 °C per minute, and incubation was incubated for 10 min. Sequences of the substrate 3 and substrate 4 were shown in Table 2.

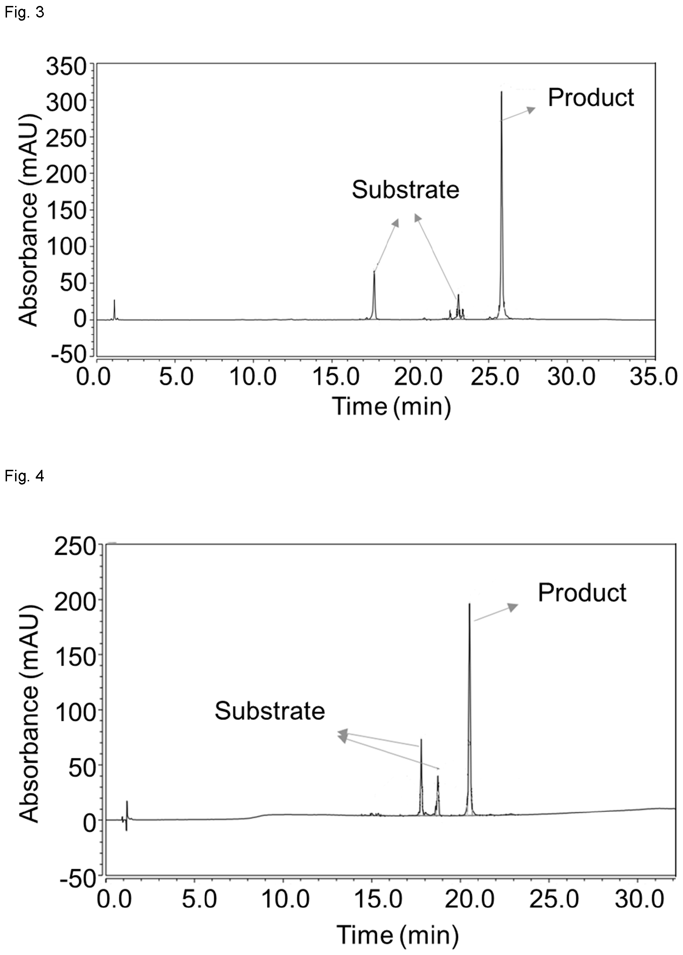

The annealed substrate 3 and substrate 4 were added to a clean reagent bottle to prepare a reaction system, causing a final volume to be 100 µL; and concentrations of the substrate 3 and substrate 4 were 100 µM, the concentration of ATP was 0.4 mM, the concentration of Tris-HCl (pH 7.5@25 °C) was 50 mM, the concentration of MgCl₂ was 10 mM, the concentration of DTT was 1 mM, and the concentration of a ligase was 0.2 mg/mL. The reaction system underwent a reaction for 16 h at 16 °C. The obtained reaction system was held for 5 min at 80 °C to deactivate the ligase, a sediment was removed through centrifugation at 12000 rpm. A product was produced through mass spectrometry identification. Results showed that a molecular weight of 24530±2 were detected in the reaction systems of the ligases L-1, L-2, L-3, L-4, L-5, L-6, L-11, L-18, L-19, L-20, L-21, L-22, and L-23, and a theoretical molecular weight was 24526±5. It indicated that a non-natural tRNA product was produced. No product molecular weight was detected in the reaction system of L-7, L-8, and L-9, indicating that no tRNA product was produced. UPLC detection was performed on the reaction system of L-1. It was found that there were new peaks generated, indicating the feasibility of the experimental protocol. UPLC results were shown in Fig. 3. UPLC detection was performed on the reaction system. It was found that a new peak with a retention time of 25.822 min was generated, and the new peak was confirmed as a product in combination with mass spectrometry results. The nick site of the above ligation was located in the stem-loop structure of the tRNA.

**Table 2**

| Serial number | Sequence | 5' end | 3' end |
|---|---|---|---|
| Substrate 3 (SEQ ID NO: 26) | 5’-mGmUmGmUmCmCmCmUmGmCmGmG mCmAmAmUmGmGmAmUmAmGmCmCmG mAmUmUmGmGmAmCmUmUmCmAmA-3 | Hydroxyl group | Hydroxyl group |
| Substrate 4 (SEQ ID NO: 27) | 5'-P- mAmUmUmCmAmAmAmGmGmUmUmC mCmGmGmGmUmUmCmGmAmGmUmCmC mCmGmGmGmGmGmAmCmGmCmG-3' | Phosphate group | Hydroxyl group |

The m before A, C, G, or U represented 2' methoxy modification for the ribonucleotide, and the P represented a phosphate group on a C atom at position 5 of a 5' end nucleotide.

The ribonucleotides of the substrate 3 and substrate 4 all had 2' methoxy modifications.

### Embodiment 4 Catalytic synthesis of non-natural tRNA using RNA ligase

A substrate 5 and a substrate 6 were dissolved in an annealing buffer (100 mM NaCl, 10 mM Tris-HCl, pH 7.5, 1 mM EDTA) based on a final concentration of 200 µM, so as to perform annealing slowly. An annealing program included: the system was heated to 90 °C and held for 1 min, and then the temperature was lowered to 20 °C with a cooling rate of 0.1 °C per minute, and incubation was incubated for 10 min. Sequences of the substrate 5 and substrate 6 were shown in Table 3.

The annealed substrate 5 and substrate 6 were added to a clean reagent bottle to prepare a reaction system, causing a final volume to be 100 µL; and concentrations of the substrate 5 and substrate 6 were 100 µM, the concentration of ATP was 0.4 mM, the concentration of Tris-HCl (pH 7.5@25 °C) was 50 mM, the concentration of MgCl₂ was 10 mM, the concentration of DTT was 1 mM, and the concentration of a ligase was 0.2 mg/mL. The reaction system underwent a reaction for 16 h at 16 °C. The obtained reaction system was held for 5 min at 80 °C to deactivate the ligase, a sediment was removed through centrifugation at 12000 rpm. A product was produced through mass spectrometry identification. Results showed that molecular weights of 24171±2 were detected in the reaction systems of the ligases L-1, L-2, L-3, L-4, L-5, L-6, L-11, L-18, L-19, L-20, L-21, L-22, and L-23, and a theoretical molecular weight was 24166±5. It indicated that a non-natural tRNA product was produced. No product molecular weight was detected in the reaction system of L-7, L-8, and L-9, indicating that no tRNA product was produced. UPLC detection was performed on the reaction system of L-1. It was found that there were new peaks generated, indicating the feasibility of the experimental protocol. UPLC results were shown in Fig. 4. UPLC detection was performed on the reaction system. It was found that a new peak with a retention time of 20.512 min was generated, and the new peak was confirmed as a product in combination with mass spectrometry results. The nick site of the above ligation was located in the stem-loop structure of the tRNA.

**Table 3**

| Serial number | Sequence | 5' end | 3' end |
|---|---|---|---|
| Substrate 5 (SEQ ID NO: 28) | 5'- mGfCmGrArCrCrUrUrArGrCrUrCrArGrUrUrGrGrUr ArG rArGrCrGrGrArGrGrArCrUrCrCfAfG-3' | Hydroxyl group | Hydroxyl group |
| Substrate 6 (SEQ ID NO: 29) | 5'-P- fAfUrCrCrUrUrArGrGrUrCrArCrUrGrGrUrUrCrGrAr A rUrCrCrGrGrUrArGrGrUrCfGfCrArCrC-3' | Phosphate group | Hydroxyl group |

The m before A, C, G, or U represented 2' methoxy modification for the ribonucleotide, the f represented 2' fluoro modification for the ribonucleotide, the r represented 2' hydroxyl modification for the ribonucleotide (unmodified ribonucleotide), and the P represented a phosphate group on a C atom at position 5 of a 5' end nucleotide.

The ribonucleotides at positions 1 and 3 of the substrate 5 had 2' methoxy modifications, the ribonucleotides at positions 2, 36 and 37 had 2' fluoro modifications, and ribonucleotides at positions 4-35 were unmodified ribonucleotides.

The ribonucleotides at positions 1, 2, 33, and 34 of the substrate 6 had 2' methoxy modifications, and the ribonucleotides at positions 3-32 and 35-37 were unmodified ribonucleotides.

### Embodiment 5 Catalytic synthesis of non-natural tRNA using RNA ligase

A substrate 7 and a substrate 8 were dissolved in an annealing buffer (100 mM NaCl, 10 mM Tris-HCl, pH 7.5, 1 mM EDTA) based on a final concentration of 200 µM, so as to perform annealing slowly. An annealing program included: the system was heated to 90 °C and held for 1 min, and then the temperature was lowered to 20 °C with a cooling rate of 0.1 °C per minute, and incubation was performed for 10 min. Sequences of the substrate 7 and substrate 8 were shown in Table 4.

The annealed substrate 7 and substrate 8 were added to a clean reagent bottle to prepare a reaction system, causing a final volume to be 100 µL; and concentrations of the substrate 7 and substrate 8 were 100 µM, the concentration of ATP was 0.4 mM, the concentration of Tris-HCl (pH 7.5@25 °C) was 50 mM, the concentration of MgCl₂ was 10 mM, the concentration of DTT was 1 mM, and the concentration of a ligase was 0.2 mg/mL. The reaction system underwent a reaction for 16 h at 16 °C. The obtained reaction system was held for 5 min at 80 °C to deactivate the ligase, a sediment was removed through centrifugation at 12000 rpm. A product was produced through mass spectrometry identification. Results showed that molecular weights of 26367±2 were detected in the reaction systems of the ligases L-1, L-2, L-3, L-4, L-5, L-6, L-11, L-18, L-19, L-20, L-21, L-22, and L-23, and a theoretical molecular weight was 26364±5. It indicated that a non-natural tRNA product was produced. No product molecular weight was detected in the reaction system of L-7, L-8, and L-9, indicating that no tRNA product was produced. The nick site of the above ligation was located in the stem-loop structure of the tRNA.

**Table 4**

| Serial number | Sequence | 5' end | 3' end |
|---|---|---|---|
| Substrate 7 (SEQ ID NO: 30) | 5'- mGsmCsmGsmAsmCsmCsmUsmUsmAsmG smCsmUsmCsmAsmGsmUsmUsmGsmGsmU smAsmGsmAsmGsmCsmGsmGsmAsmGsmG smAsmCsmUsmCsmCsmAsmG-3' | Hydroxyl group | Hydroxyl group |
| Substrate 8 (SEQ ID NO: 31) | 5'-sP-mAsmUsmCsmCsmUsmUsmAsmGsmG smUsmCsmAsmCsmUsmGsmGsmUsmUsmC smGsmAsmAsmUsmCsmCsmGsmGsmUsmA smGsmGsmUsmCsmGsmCrArCrC-3 | Phosphate group | Hydroxyl group |

The m before A, C, G, or U represented 2' methoxy modification for the ribonucleotide, the r represented 2' hydroxyl modification for the ribonucleotide (unmodified ribonucleotide), the s represented phosphorothioate modification, and the P represented a phosphate group on a C atom at position 5 of a 5' end nucleotide.

All the ribonucleotides of the substrate 7 had 2' methoxy modifications, and the ribonucleotides at positions 2-37 had the phosphorothioate modifications.

The ribonucleotides at positions 1-35 of the substrate 8 had 2' methoxy modifications, the ribonucleotides at positions 1-35 had phosphorothioate modifications, and the ribonucleotides at positions 36-38 were unmodified ribonucleotides.

### Embodiment 6 Catalytic synthesis of natural tRNA using RNA ligase

A substrate 9 and a substrate 10 were dissolved in an annealing buffer (100 mM NaCl, 10 mM Tris-HCl, pH 7.5, 1 mM EDTA) based on a final concentration of 200 µM, so as to perform annealing slowly. An annealing program included: the system was heated to 90 °C and held for 1 min, and then the temperature was lowered to 20 °C with a cooling rate of 0.1 °C per minute, and incubation was performed for 10 min. Sequences of the substrate 9 and the substrate 10 were shown in Table 5.

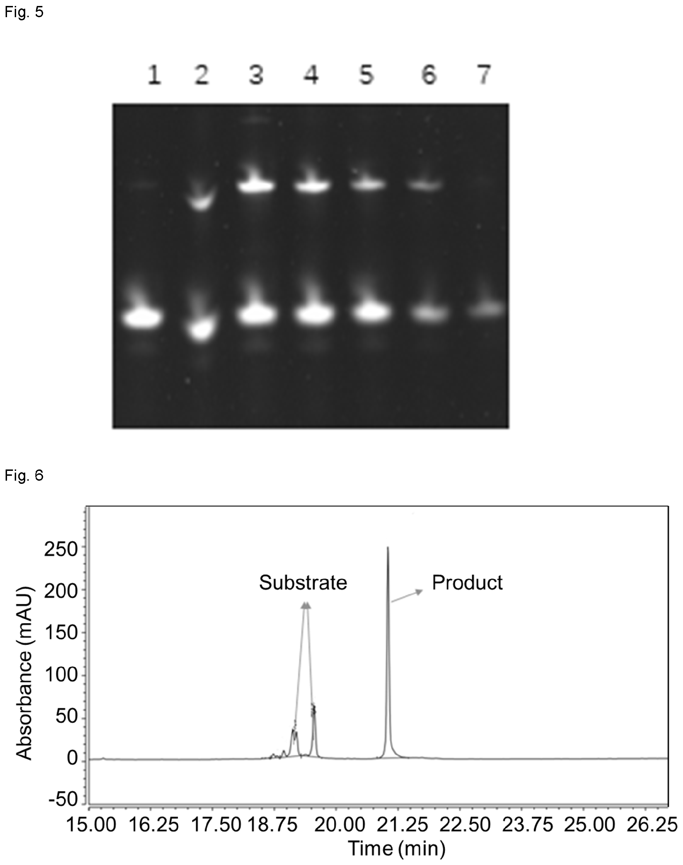

The annealed substrate 9 and substrate 10 were added to a clean reagent bottle to prepare a reaction system, causing a final volume to be 100 µL; and concentrations of the substrate 9 and substrate 10 were 100 µM, the concentration of ATP was 0.4 mM, the concentration of Tris-HCl (pH 7.5@25 °C) was 50 mM, the concentration of MgCl₂ was 10 mM, the concentration of DTT was 1 mM, and the concentration of a ligase was 0.2 mg/mL. The reaction system underwent a reaction for 16 h at 16 °C. The obtained reaction system was held for 5 min at 80 °C to deactivate the ligase, a sediment was removed through centrifugation at 12000 rpm. A product was produced through mass spectrometry identification. Results showed that molecular weights of 23560.3 were detected in the reaction system of the ligases L-1, L-2, L-3, L-4, L-5, L-6, L-10, L-11, L-12, L-13, L-14, L-15, L-16, and L-17, and a theoretical molecular weight was 23564±5. It indicated that a natural tRNA product was produced. No product molecular weight was detected in the reaction system of L-7, L-8, and L-9, indicating that no tRNA product was produced. PAGE gel detection was performed on the above partial reaction sample. An electrophoresis result diagram was shown in Fig. 5. It was found that new bands were generated in the reaction system, while no bands were generated in the system where no product was detected by mass spectrometry, consistent with the band of the negative control reaction without adding the enzyme. The nick site of the above ligation was located in the complementary region of the tRNA. Lane 1 in Fig. 5 was a negative control group without adding the ligase, Lane 2 was a reaction system that was catalyzed using the ligase L-10, Lane 3 was a reaction system that was catalyzed using the ligase L-11, Lane 4 was a reaction system that was catalyzed using the ligase L-12, Lane 5 was a reaction system that was catalyzed using the ligase L-1, Lane 6 was a reaction system that was catalyzed using the ligase L-2, and Lane 7 was a reaction system that was catalyzed using the ligase L-7.

**Table 5**

| Serial number | Sequence | 5' end | 3' end |
|---|---|---|---|
| Substrate 9 (SEQ ID NO: 32) | 5’- GUGUCCCUGCGGCAAUGGAU AGCCGAUUGGACUUCAAAUU- 3' | Hydroxyl group | Hydroxyl group |
| Substrate 10 (SEQ ID NO: 33) | 5’-P- CAAAGGUUCCGGGUUCGAG UCCCGGGGGACGCG-3’ | Phosphate group | Hydroxyl group |

The P represented a phosphate group on a C atom at position 5 of a 5' end nucleotide.

### Embodiment 7 Catalytic synthesis of non-natural tRNA using RNA ligase

A substrate 11 and a substrate 12 were dissolved in an annealing buffer (100 mM NaCl, 10 mM Tris-HCl, pH 7.5, 1 mM EDTA) based on a final concentration of 200 µM, so as to perform annealing slowly. An annealing program included: the system was heated to 90 °C and held for 1 min, and then the temperature was lowered to 20 °C with a cooling rate of 0.1 °C per minute, and incubation was performed for 10 min. Sequences of the substrate 11 and substrate 12 were shown in Table 6.

The annealed substrate 11 and substrate 12 were added to a clean reagent bottle to prepare a reaction system, causing a final volume to be 100 µL; and concentrations of the substrate 11 and substrate 12 were 100 µM, the concentration of ATP was 0.4 mM, the concentration of Tris-HCl (pH 7.5@25 °C) was 50 mM, the concentration of MgCl₂ was 10 mM, the concentration of DTT was 1 mM, and the concentration of a ligase was 0.2 mg/mL. The reaction system underwent a reaction for 16 h at 16 °C. The obtained reaction system was held for 5 min at 80 °C to deactivate the ligase, a sediment was removed through centrifugation at 12000 rpm. A product was produced through mass spectrometry identification. Results showed that molecular weights of 24601.6 were detected in the reaction systems of the ligases L-1, L-2, L-3, L-4, L-5, L-6, L-10, L-11, L-12, L-13, L-14, L-15, L-16, and L-17, and a theoretical molecular weight was 24606.2±5. It indicated that a non-natural tRNA product was produced. No product molecular weight was detected in the reaction system of L-7, L-8, and L-9, indicating that no tRNA product was produced. UPLC was performed on the reaction system of L-1 to detect the generation of a product. Results showed that there were new peaks generated, indicating the technology was feasible. UPLC results were shown in Fig. 6. UPLC detection was performed on the reaction system. It was found that a new peak with a retention time of 21.045 min was generated, and the new peak was confirmed as a product in combination with mass spectrometry results. The nick site of the above ligation was located in the complementary region of the tRNA.

**Table 6**

| Serial number | Sequence | 5' end | 3' end |
|---|---|---|---|
| Substrate 11 (SEQ ID NO: 34) | 5'- mGmUmGmUmCmCmCmUmGmCmGmGmCmA mAmUmGmGmAmUmAmGmCmCmGmAmUmU mG mGmAmCmUmUmCmAmAmAmUmU-3' | Hydroxyl group | Hydroxyl group |
| Substrate 12 (SEQ ID NO: 35) | 5'-P- mCmAmAmAmGmGmUmUmCmCmGmGmGmU mUmCmGmAmGmUmCmCmCmGmGmGmGmG mA mCmGmCmG-3' | Phosphate group | Hydroxyl group |

The m before A, C, G, or U represented 2' methoxy modification for the ribonucleotide, and the P represented a phosphate group on a C atom at position 5 of a 5' end nucleotide.

The ribonucleotides of the substrate 11 and substrate 12 all had 2' methoxy modifications.

### Embodiment 8 Catalytic synthesis of non-natural tRNA using RNA ligase

More fragments were attempted for tRNA synthesis. Substrates 13, 14 and 15 were dissolved in an annealing buffer (100 mM NaCl, 10 mM Tris-HCl, pH 7.5, 1 mM EDTA) based on a final concentration of 300 µM, so as to perform annealing slowly. An annealing program included: the system was heated to 90 °C and held for 1 min, and then the temperature was lowered to 20 °C with a cooling rate of 0.1 °C per minute, and incubation was performed for 10 min. Sequences of the substrates 13, 14 and 15 were shown in Table 7.

The annealed substrates 13, 14 and 15 were added to a clean reagent bottle to prepare a reaction system, causing a final volume to be 100 µL; and concentrations of the substrates 13, 14 and 15 were 100 µM, the concentration of ATP was 0.4 mM, the concentration of Tris-HCl (pH 7.5@25 °C) was 50 mM, the concentration of MgCl₂ was 10 mM, the concentration of DTT was 1 mM, and the concentration of a ligase was 0.2 mg/mL. The reaction system underwent a reaction for 16 h at 16 °C. The obtained reaction system was held for 5 min at 80 °C to deactivate the ligase, a sediment was removed through centrifugation at 12000 rpm. A product was produced through mass spectrometry identification. Results showed that molecular weights of 24601.6 were detected in the reaction systems of the ligases L-1, L-2, and L-4, and a theoretical molecular weight was 24606.2±5. It indicated that a non-natural tRNA product was produced. The nick sites of the above ligations were located in the stem-loop structure of the tRNA.

**Table 7**

| Serial number | Sequence | 5' end | 3' end |
|---|---|---|---|
| Substrate 13 | 5'-mGmUmGmUmCmCmCmUmGmCmG | Hydroxyl | Hydroxyl |
| (SEQ ID NO: 36) | mGmCmAmAmUmGmGmAmUmAmGmC mCmGmAmUmUmGmGmAmCmUmUmC mAmAmAmUmU-3' | group | group |
| Substrate 14 (SEQ ID NO: 37) | 5'-P- mAmUmUmCmAmAmAmGmGmUmU mCmCmGmGmGmUmUmC-3' | Phosphate group | Hydroxyl group |
| Substrate 15 (SEQ ID NO: 38) | 5'-P- mGmAmGmUmCmCmCmGmGmGmG mGmAmCmGmCmG-3' | Phosphate group | Hydroxyl group |

The m before A, C, G, or U represented 2' methoxy modification for the ribonucleotide, and the P represented a phosphate group on a C atom at position 5 of a 5' end nucleotide.

The ribonucleotides of the substrate 13, substrate 14, and substrate 15 all had 2' methoxy modifications.

It may be seen from the above description that, in the above embodiments of the present disclosure, the following technical effects are realized. In the present disclosure, it is found that some RNA ligase can achieve the biosynthesis of a non-natural tRNA containing non-natural nucleotides, and can be used for the biosynthesis of the tRNA and even the non-natural tRNA. With the method, the tRNA can be prepared efficiently, the yield and purity of the product are high, and non-natural tRNA that is difficult to synthesize in the prior art can be prepared.

The above are only the preferred embodiments of the present disclosure and are not intended to limit the present disclosure. For person skilled in the art, the present disclosure may have various modifications and variations. Any modifications, equivalent replacements, improvements and the like made within the spirit and principle of the present disclosure all fall within the scope of protection of the present disclosure.

## Claims

1. A method for biosynthesizing a tRNA, wherein the method comprises:
ligating the 3'end and 5' end of different substrates to form a phosphodiester bond with an RNA ligase, thereby forming the tRNA, wherein the number of the substrates is ≥ 2;
the RNA ligase comprises any one or more enzymes in the RNA ligase family Rnl1, Rnl2, Rnl3 or Rnl5.

2. The method according to claim 1, wherein the substrates comprise one or more non-natural nucleotides, and the tRNA comprise a non-natural tRNA.

3. The method according to claim 2, wherein the RNA ligase comprises:
one or more of the RNA ligases as shown in SEQ ID NO: 1, SEQ ID NO: 2, SEQ ID NO: 3, SEQ ID NO: 4, SEQ ID NO: 5, SEQ ID NO: 6, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22 or SEQ ID NO: 23; or
a protein having more than 70% identity with any of the RNA ligases as shown in SEQ ID NO: 1 to SEQ ID NO: 6, or SEQ ID NO: 10 to SEQ ID NO: 23 and having an RNA ligase activity.

4. The method according to claim 2, wherein the non-natural tRNA comprises a stem-loop structure and a complementary region, and a ligation site of the substrates is located at the stem-loop structure and/or the complementary region.

5. The method according to claim 2, wherein the 3' ends of the substrates are hydroxyl groups.

6. The method according to claim 2, wherein the substrates comprise a first substrate and other substrates, wherein the 5' end of the first substrate forms the 5' end of the non-natural tRNA, and the 5' ends of the other substrates are phosphate groups.

7. The method according to claim 2, wherein the number of the non-natural nucleotides in the substrates is ≥ 2.

8. The method according to claim 7, wherein the nucleotides at the 5' ends and/or 3' ends of the substrates are the non-natural nucleotides.

9. The method according to claim 8, wherein the substrates consist of the non-natural nucleotides.

10. The method according to claim 2, wherein the number of the substrates is 2 to 6; and
the length of each of the substrates is 15 nt to 60 nt.

11. The method according to claim 2, wherein the method comprises: substrate annealing and substrate ligation;
the substrate annealing comprises: dissolving the substrates in an annealing buffer to form an annealing system, annealing the annealing system according to an annealing program, wherein the annealing program comprises: heating the annealing system to 90°C and holding for 1 minute, and then lowering the temperature to 20°C with a cooling rate of 0.1°C per minute and incubating for 10 minutes to obtain an annealing product; and
the substrate ligation comprises: formulating the annealing product, the RNA ligase, and an ATP into a ligation system for the substrate ligation.

12. The method according to claim 11, wherein
the annealing buffer comprises: 1 mM to 10 M of NaCl, 1 mM to 10 M of Tris-HCl at pH 4 to 10 and 0.1 mM to 10 M of EDTA;
a reaction temperature for the substrate ligation is 0°C to 70°C, and a reaction time for the substrate ligation is 10 minutes to 100 hours; and
a concentration of the annealing product is 10 µM to 2 mM, a concentration of the RNA ligase is 0.02 mg/mL to 4 mg/mL, and a molar ratio of the ATP to the annealing product is 1: 1 to 10: 1.

13. The method according to claim 12, wherein
the ligation system further comprises MgCl₂ and dithiothreitol, wherein,
a concentration of MgCl₂ is 0.1 mM to 200 M, and a concentration of dithiothreitol is 0.1 mM to 10 M.

14. The method according to claim 2, wherein the non-natural nucleotide comprises a ribonucleotide having one or more of the following modifications: a ribose position 2' modification, a ribose skeleton modification, a base modification or a phosphate skeleton modification;
the ribose position a 2' modification comprises a 2'-methoxy modification, a 2'-fluoro modification, a 2'-methoxyethyl modification, a 2'-FANA modification, a locked nucleic acid modification or a hexitol nucleic acid modification;
the ribose skeleton modification comprises replacing the ribose in the nucleotide with ribuloNA, TNA, tPhoNA or dXNA;
the base modification comprises a denitrified adenine C7 modification, a denitrified guanosine C7 modification, a cytosine C5 modification, or a uridine C5 modification; and
the phosphate skeleton modification comprises a PS modification.

15. A tRNA, wherein the tRNA comprises the tRNA obtained with the method of any one of claims 1 to 14.

16. The tRNA according to claim 15, wherein the tRNA comprises the non-natural tRNA obtained with the method of any one of claims 2 to 14.
